# EUROPEAN PATENT SPECIFICATION

(11) **EP 4 303 575 B1**
(45) Date of publication and mention of the grant of the patent: **03.09.2025**
(21) Application number: 22183742.0
(22) Date of filing: 08.07.2022
(51) Int. Cl.: G01N 23/18, G01N 33/12

(54) **METHOD OF VERIFYING THE DETECTION CAPABILITY OF AN X-RAY INSPECTION APPARATAUS**
VERFAHREN ZUR VERIFIZIERUNG DER DETEKTIONSFÄHIGKEIT EINER RÖNTGENPRÜFVORRICHTUNG
PROCÉDÉ DE VÉRIFICATION DE LA CAPACITÉ DE DÉTECTION D'UN APPAREIL D'INSPECTION À RAYONS X

(43) Date of publication of application: 10.01.2024
(73) Proprietor: Mettler-Toledo, LLC, Columbus OH 43240 (US)
(72) Inventor: MAHNKE, Guido, 65201 Wiesbaden (DE); HOFFMANN, Benedikt, 65510 Idstein (DE)
(74) Representative: Leinweber & Zimmermann

(56) References cited:
- US-A- 5 095 499
- N.J.B. MCFARLANE ET AL: "Detection of Bone Fragments in Chicken Meat using X-ray Backscatter", BIOSYSTEMS ENGINEERING, vol. 85, no. 2, 1 June 2003 (2003-06-01), AMSTERDAM, NL, pages 185 - 199, XP055356395, ISSN: 1537-5110, DOI: 10.1016/S1537-5110(03)00036-9

## Description

The invention is in the field of X-ray inspection of products, in particular of food products. In particular, the invention relates to a method of verifying the detection capability of an X-ray inspection apparatus with respect to a product type of in particular a food product, wherein a body is subject to X-rays propagating through the body in an inspection zone of the X-ray inspection apparatus.

X-ray inspection by X-ray inspection apparatus is widely known in the field of food quality control and/or food analysis, f.i. to determine the meat and fat content of food items such as meat items. For instance, US 6,597,759 B2 discloses the non-destructive analysis of products by using dual-energy X-ray attenuation measurements to determine the composition of bone in meat products. Thereto, a beam of X-ray radiation having first and second energies is generated and a meat sample of arbitrary size is inserted into the beam. The attenuation of the X-ray beam at the first and second energies is detected after a test passed through the meat sample. From a previously determined index of photoelectric absorption and Compton scattering values corresponding to meat and bone, and the attenuation of the X-rays at the first and second energies, a ratio of bone and non-bone portions of meat is deduced. For calibration, a phantom having a known composition and known X-ray attenuation characteristic is used. The advantage of using a phantom instead of meat products themselves for calibration purposes is, of course, to have a non-decaying calibration body usable over longer time periods.

However, it turned out that even using phantoms somehow mimicking by material selection/material composition a product sample of the respective meat product, changes in detection capability of X-ray inspection apparatus may remain undetected notwithstanding calibration at usual time intervals, and in use such apparatus may let pass food products with undetected, undesired and maybe even dangerous bone pieces.

D1, N.J.B. MCFARLANE ET AL: "Detection of Bone Fragments in Chicken Meat using X-ray Backscatter", BIOSYSTEMS ENGINEERING, vol. 85, no. 2, 1 June 2003 (2003-06-01), pages 185-199, XP055356395, AMSTERDAM, NL, ISSN: 1537-5110, DOI: 10.1016/S1537-511(03)00036-9, discloses a phantom comprised of a polystyrene cover and a polystyrene block having fragments of chicken bones inserted in slots of the polystyrene block to be compared with real pieces of chicken breast containing bone fragments just below the surface, and provides analysis about energy windows thereto.

US 5,095,499 A discloses a radiological phantom for use in X-ray mammography, being composed of a bottom plate including a step wedge section, a spacer and a cover plate superposed with one another and orienting means in form of orienting means providing that test objects will lie in predetermined orientations relative to one another in the imaging plane when the body is oriented by the orienting means.

The object of the present invention is to improve methods of verifying the detection capability of X-ray inspection apparatus as initially mentioned, in particular aiming at higher reliability of said apparatus in intended use.

To this end, the invention provides a further development of such methods as set out initially, in accordance with all features of claim 1. It is provided, among other, that the body is a phantom mainly made from artificial material and comprising at least two regions with different propagation path lengths, their path length difference correlating with a difference of measured X-ray attenuation arising between regions of a product sample of said product type mimicked by the phantom which regions correspond to said regions of the phantom.

In the framework of the invention it was recognized that using conventional phantoms for calibration or verifying the detection capability is no sufficient challenge for X-ray apparatus since internal structure/composition information is lost with respect to the original product sample. By the inventive method, said challenge is increased, such that X-ray inspection apparatus having their detection capabilities verified by the inventive method are better exercised regarding their task, as f.i. detecting also small pieces of bone in poultry meat products in particular within regions with high X-ray attenuation if no bone particle would be present.

The method is preferably applied for product types with inhomogeneous internal structure as poultry meat, in particular chicken breast, but can be applied also to other meat products, food products in general and even products outside the field of food products where the product type is of inhomogeneous internal structure.

In a preferred embodiment, the phantom material comprises a polymer material as base material. It is understood that the polymer material used should have preferably an absorption characteristic similar/close to the main material of the product type, for the above example poultry meat (meaning that high- and low-energy X-rays should be absorbed in a similar relation as said f.i. poultry meat). In this regard, it is preferred that the polymer material comprises epoxy resin or is made thereof, wherein also a blend of several epoxy resins can be provided. Also envisaged are materials comprising/consisting of PMMA can be suitable, since their primary elements have similar effect in the X-ray image as poultry meat. Polymer materials as polyethylene are thinkable but less preferred. Further, in preferred embodiments, polymers comprising fluorine as PTFE or also PVC are not contained in the material.

In a further preferred embodiment, the phantom comprises locally a piece of a different material than said base material, in particular a calcium containing material, preferably a calcium sulfate or calcium phosphate, in particular in the region having the longer prolongation path length. Such piece, mimicking bone, is, thus, comprised in the phantom to imitate a case which is among those most critical to pass undetected by X-ray inspection when arising for a natural product subject to inspection, namely (in particular small/thin) bone pieces in particular in a region providing high attenuation already in the natural product, which regions corresponding to those in the phantom with long propagation path (irrespective of the propagation path length through the natural product). An areal extension transverse to the propagation direction of at least one piece is preferably lower than 20 mm², preferably than 13 mm², in particular than 10 mm², even than 8 mm², even than 6 mm². In a further preferred embodiment, a transverse dimension of one or more pieces is, by means of a factor in the range of 2.5 to 7.5, larger than the pixel extension of the detector resolution of the apparatus.

This aspect of the phantom to mimic bone in a localized manner is also disclosed alone-standing as protection-worthy. Accordingly, the invention also provides a phantom to be used in a method of verifying the detection capability of an X-ray inspection apparatus with respect to a product type of in particular a food product, the phantom being mainly made from artificial material and comprising locally a piece of a different material than said polymer material in form of a calcium containing material. It is understood that this independent aspect of the present invention can be provided also in combination with the above-described and subsequently described features.

Further preferred, the phantom comprises a plurality of pieces of different material, said plurality in particular comprising a first group of pieces varying in their dimension in propagation path direction, a second group of pieces varying in dimension and/or form transverse to the propagation path direction, and/or a third group of pieces varying in material composition. Having f.i. a plurality of such pieces with f.i. different thicknesses, a scaling grade is provided which can be used for checking the limits of detection capability of the verified X-ray inspection apparatus. In a particularly preferred embodiment, a piece with a first thickness (e.g. dimension in propagation path direction) is provided, corresponding to a first detection limit of the X-ray inspection apparatus, having the purpose that the setting of the X-ray inspection apparatus is chosen to no more recognize the detection thereof as criterion for sorting out. Further preferred is a piece having a second thickness corresponding to a setting of the inspection apparatus in which said piece is reliably recognized in X-ray inspection of the phantom. There can be intermediate piece thicknesses to create a finer-graded scale, or also thicker pieces to gain information about the grade of deterioration of the detection capability. These pieces are at least in part preferably arranged in not-overlapping positions with respect to the X-ray propagation.

In a preferred embodiment, the phantom locally comprises such a piece with a ratio of propagation path length of the piece to overall propagation path length of less than 7%, more preferably less than 5.6%, in particular less than 4.2%, even less than 3%. On the other hand side, it is preferred that the phantom locally comprises such a piece with a ratio of propagation path length in the piece to overall propagation path length in the phantom of higher than 2.4%, more preferred higher than 3.6%, in particular higher than 4.2%, even higher than 4.8%, and preferably thicker than the earlier mentioned piece.

In a further preferred embodiment, alternatively or additionally, a similar piece is also contained preferably in a region with lower path length, leading to a ratio of less than 12%, preferably less than 10%, in particular less than 8%, and/or higher than 6%, preferably higher than 10%, in particular higher than 14%. These ranges may, depending on the product types, also apply to the region with higher/highest propagation path length.

Other pieces can be included, f.i. from metal, to allow additional verification check for the apparatus with respect to other verification containing simple phantoms with metal tags, f.i. for reference purposes.

Variations in form may mimic typical bone forms which may arise in poultry meat products, such as f.i. a fan bone, a wish bone, and/or a rib bone. In a preferred embodiment, one, some or all of said locally arranged pieces is/are embedded in the base material. This again better mimics the true situation of product samples faced by the X-ray inspection apparatus with respect to said product type. Moreover, duration of the phantoms is increased. Further, it is envisaged that bone (splitter) mimics are embedded in different height levels, in view of consideration of defined focus plane of some apparatus type.

In a further preferred embodiment, the overall area shape of the product sample transverse to the propagation path is mimicked by the phantom. That is, the contour of the phantom corresponds to a typical contour of the product type, while the height profile is not mimicking the thickness of the product sample but relates to the X-ray attenuation of the various regions of the product sample. In this regard, the path length difference discussed above with respect to at least two regions preferably applies to the overall phantom body having, thus, a height profile correlating not directly with the thickness profile of the product sample but directly correlating with the respective local attenuation of the product sample.

In a preferred embodiment, the apparatus is capable of dual-energy X-ray attenuation measurement.

Such dual-energy X-ray apparatus are capable of taking simultaneously an image at lower and an image at higher X-ray energy. By suitable evaluation algorithms, information from both images taken can be combined to create an image effectively eliminating the meat part for easier localization of paths corresponding to higher attenuation which likely can be bone pieces or bone parts. Single-energy X-ray apparatus would already have problems in the detection of bone pieces at all in materials having inhomogeneous thickness. The evaluation algorithms may include to be sensitive to the local gradient of attenuation and/or to the overall area of higher attenuation regions.

Accordingly, X-ray inspection of the phantom generates a realistic X-ray image. Several difficulties arising in the X-ray detection of product samples are reconstructed, allowing a more reliable determination whether the X-ray apparatus achieves a desired detection capability.

In a preferred application, the method is used just aiming at verification, whether the detection capability of the verified X-ray inspection apparatus is within given set detection parameters. Moreover, such verification is intended also as continuously repeated verification, that is the X-ray inspection is repeated on the same X-ray inspection machine after a time interval. Moreover, the detection performance of a plurality of X-ray inspection apparatus can be compared between each other by executing the method with the same (or identically manufactured) phantom on another X-ray inspection apparatus of in particular the same apparatus type.

All these aspects may firstly do not require any consequences and serve for information purposes. However, in a further aspect of the invention it is also envisaged that settings of the X-ray inspection apparatus are adjusted in dependency of the result of the X-ray inspection of the phantom. In a further application aspect, the phantom may also be used as calibration phantom.

As initially said, the aim of the invention comprises to enhance the safety aspect regarding X-ray inspected products. Accordingly, the invention encompasses also a method of X-ray inspection of productions, in particular food products, more specifically poultry meat products, of a given product type by means of an X-ray inspection apparatus being in particular configured to perform dual-energy X-ray attenuation measurement, which is characterized in that said X-ray inspection apparatus is made subject to a verifying method according to any of the aspects and features discussed above with respect to said given product type.

Further, the invention provides a method of manufacturing a phantom for use in a method according to any of the previous aspects of the disclosed verifying method, < namely a method in accordance with all features of claim 11, providing among other that > an X-ray measurement for a product sample of said product type is made, the respective local measured X-ray attenuation is converted into a thickness profile having thickness variations correlated to respective X-ray attenuation variations of the product sample, and manufacturing the phantom with a thickness in dependency of said thickness profile. That is, at least partly an X-ray optical density profile is converted into a thickness profile. Further, it is preferred that the energy level used for the X-ray image for the phantom manufacturing differs from the lower energy level of the verified dual X-ray apparatus by less than 80%, preferably less than 60% of the energy level difference of said apparatus.

The thickness profile can be generated in a way that it corresponds to a height profile of the phantom, that is the phantom is made by having in a preferred manner a planar bottom side. This makes the phantom easier to handle in application. In a further preferred development of such method, the method comprises determining an average absorption coefficient by use of an average thickness of the measured product sample and an average over the intensity of the transmitted X-ray, and calculating a local thickness, in particular pixelwise with respect to the X-ray image, by means of the average absorption coefficient and the local intensity of the corresponding local X-ray intensity of the measured product sample, to obtain relative thickness differences.

In this connection, to still better mimicking the product sample in dependency of the material used for the phantom, it is in a preferred way provided that determination of the thickness profile for the phantom involves a global scaling of said relative thickness variations in dependency of the absorption coefficient of the polymer material used as base material for the phantom.

In a further preferred method aspect, the manufacturing of the phantom involves casting a polymer material into a mold, the mold form being created in particular on the basis of 3-D printing. However, other manufacturing methods can be used, f.i. a 3-D printing (f.i. FDM/FFF, SLS) of the phantom itself (and not only of its negative for the mold).

The verification method and phantom of the present invention has already advantages even if no additional material mimicking bone is provided. Such applications may in particular be useful in the framework of X-ray inspection of meat pieces where bone has been previously removed ("mechanical deboning"), since such meat would still not be comprised of homogenous meat but may comprise inhomogenities by residual elements even if not being bone, also those residuals representing impurities with effect to the detection capability of X-ray inspection apparatus aiming at detection of unintended remaining bone material, such impurities being included when basing the phantom on not the thickness of the product sample but on an X-ray image of the product sample, according to the main aspect of the present invention. However, in a preferred embodiment, the phantom is provided with the locally provided, in particular embedded pieces of different material.

In terms of using a casting process, it is preferred in this regard that the casting is divided in two or more steps and one or more pieces of a different material, in particular calcium containing material is inserted inbetween two casting steps.

These aspects of manufacturing a phantom are also disclosed in terms of system aspects. Such system to provide for a method of manufacturing a phantom according to any of the preceding aspects comprises a system control configured to create from a given product sample an X-ray image and to control manufacturing of the phantom such that its thickness profile is based on said X-ray image of the product sample. Here, of course, the product sample is the true product which could be a chicken breast or other poultry meat product, or other products as mentioned above. Further, the invention provides a phantom mimicking a product type of an in particular food product for a verifying method according to any of the precedingly discussed aspects and/or manufactured by a method according to any of the precedingly discussed aspects , <the phantom having all features of claim 16.>

Further details, features and advantages of the invention can be taken from the subsequent description of embodiments on the basis of the figures, wherein:
Fig. 1 shows a height profile of a phantom mimicking a meat product,
Fig. 2 shows a phantom with a height profile corresponding to that of Fig. 1, with additional pieces mimicking bone,
Fig. 3 shows a phantom with a height profile corresponding to that of Fig. 1, with additional pieces mimicking bone pieces,

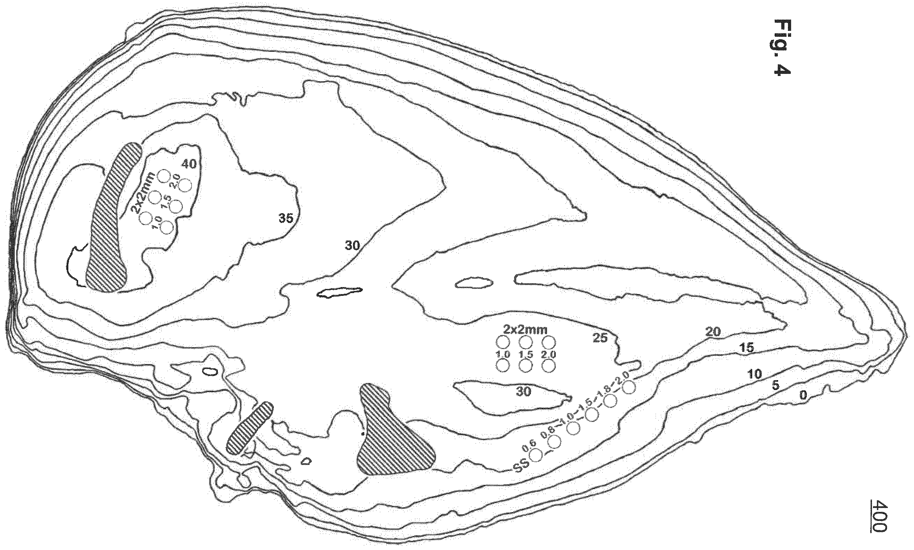

Fig. 4 shows an embodiment combining Figs. 2 and 3,
Fig. 5 shows an X-ray inspection apparatus in a schematical manner, and
Fig. 6 shows a flow diagram of a method to manufacture a phantom.

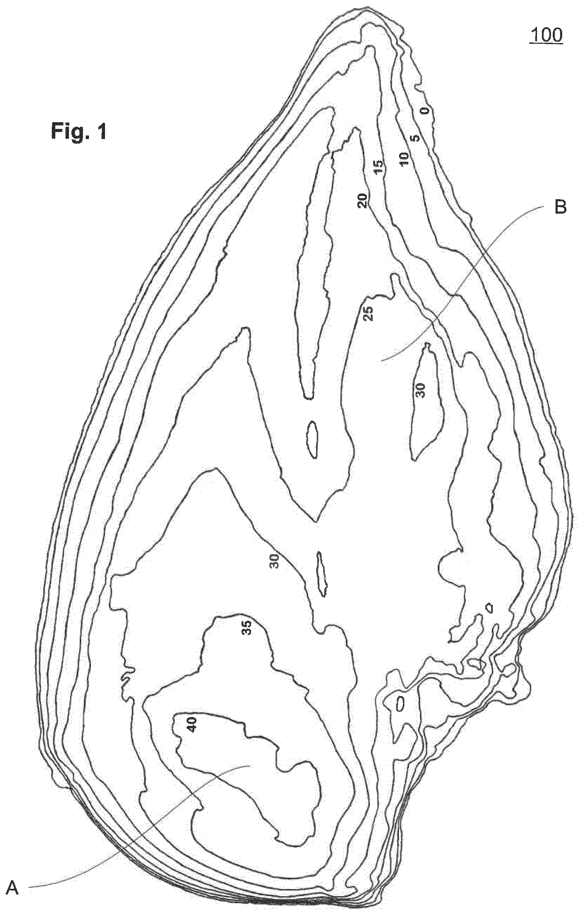

The phantom 100 shown in Fig. 1 is made from epoxy resin, the form of the phantom 100 being determined via a mold in which the phantom is casted. One recognizes from Fig. 1 that the contour of the phantom 100 is mimicking the contour of a typical product sample of a chicken breast meat product. The numbers within the outer contour of the phantom 100 are no reference numerals but numerals indicating the height profile of the phantom 100 with respect to its bottom surface, which is planar in the subject embodiment. One recognizes from Fig. 1 f.i. in the left part, several plateaus having 30 mm, 35 mm, and 40 mm (region A) as height levels, while in the right side there are several plateaus having lower height levels of in this embodiment 25 mm (region B), 20 mm, and 15 mm or lower. These height levels do not match the height level/thickness of a product sample of the product type mimicked by the phantom 100, but are based on an X-ray image of such a product sample. To obtain said height profile, the following method was applied in the subject embodiment. First, an X-ray image was taken from a selected product sample, giving the outer contour and local distribution of the X-ray attenuation. By making use of the absorption formula I=I₀ · exp (-µ · d), one can derive an average formula I_{avg} = I₀. exp (- µ_{avg} · d_{avg}), where I₀ is the intensity of the initial X-ray, I is the intensity of the X-ray transmitted through the product sample, and the averages refer to the average thickness of the probe and the average of the X-ray in the overall image. Having therefrom established µ_{avg}, one may calculate a relative local thickness, pixelwise (for every value obtained from the X-ray image according to the scaling of the X-ray image), thereby arriving for each pixel in the image, respectively region of pixels depending on the desired resolution, a thickness information reflecting the relative differences with respect to the X-ray image of the product sample.

By X-ray measurement of the absorption coefficient of the material used for manufacturing the phantom, respectively from such known data a global factor to be multiplied with the thickness values if applied, such that the absolute absorption of the original image matches the absorption of the phantom.

Accordingly, the height profile of the phantom 100 is based on the X-ray image of the product sample and reproduces the X-ray image locally when itself subject to X-ray inspection via the resulting different propagation path lengths correlating with the difference of measured X-ray attenuation arising between respective corresponding regions of the product sample.

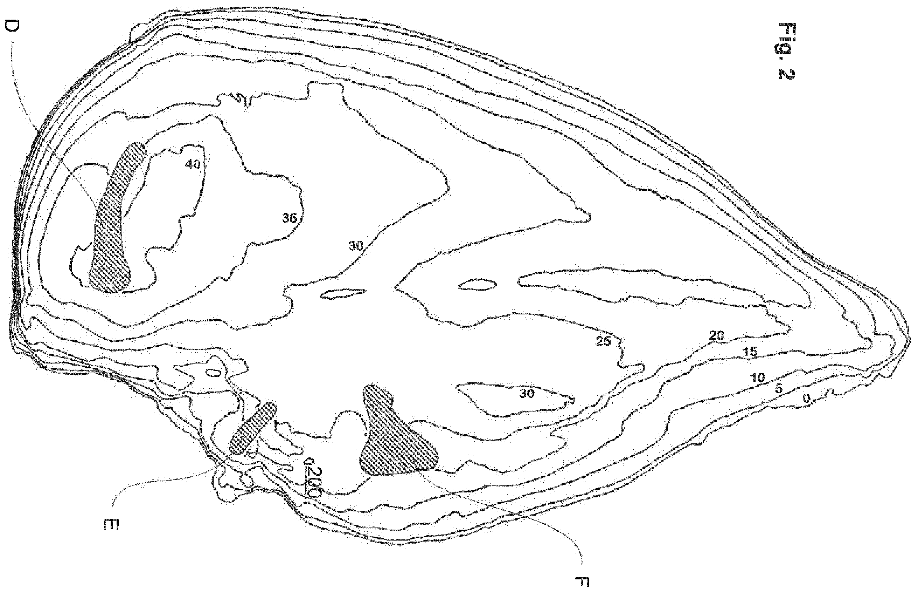

The embodiment of Fig. 2 is similar to that of Fig. 1, however, in portions D, E, and F3, the resin material is replaced by a calcium containing material, in the present embodiment plaster, to mimic typical forms of bones (BM) which could appear in such product types. In the present embodiment, BM in D mimics a wish bone, BM in E mimics a rib bone, and BM in F mimics a fan bone.

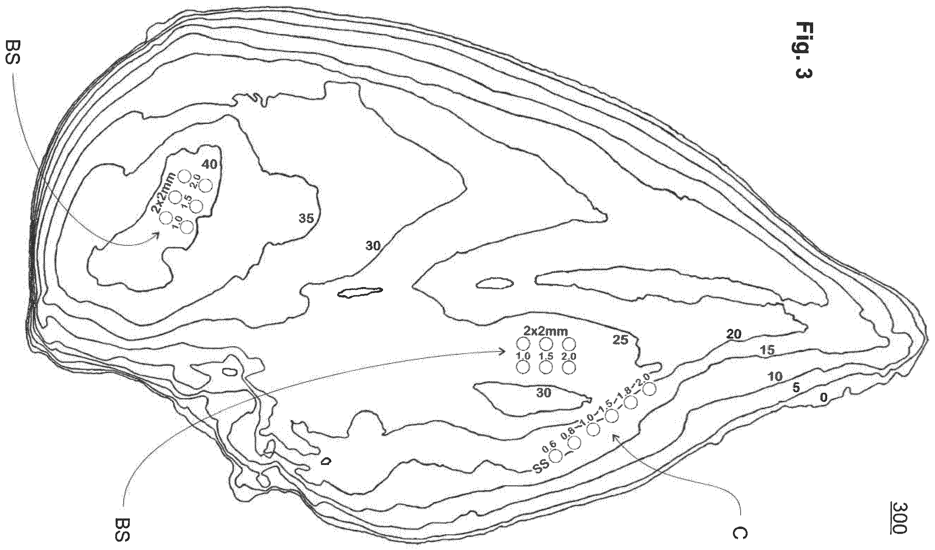

In the embodiment of Fig. 3, pieces of calcium containing material, here made of plaster, are embedded in phantom 300 as displayed in the region A and region B. These pieces mimic bone splitter or small bone pieces and are provided in region A in two rows with increasing thickness, from 1.0 mm to 2.0 mm, with each an area of 2 x 2 mm. The same set of bone splitter mimicking material pieces is present in region B. From X-ray inspection of said phantom 300, one recognizes whether the detection capability is sufficient to identify these bone splitter mimics, respectively where the detection limit of the X-ray inspection apparatus is reached.

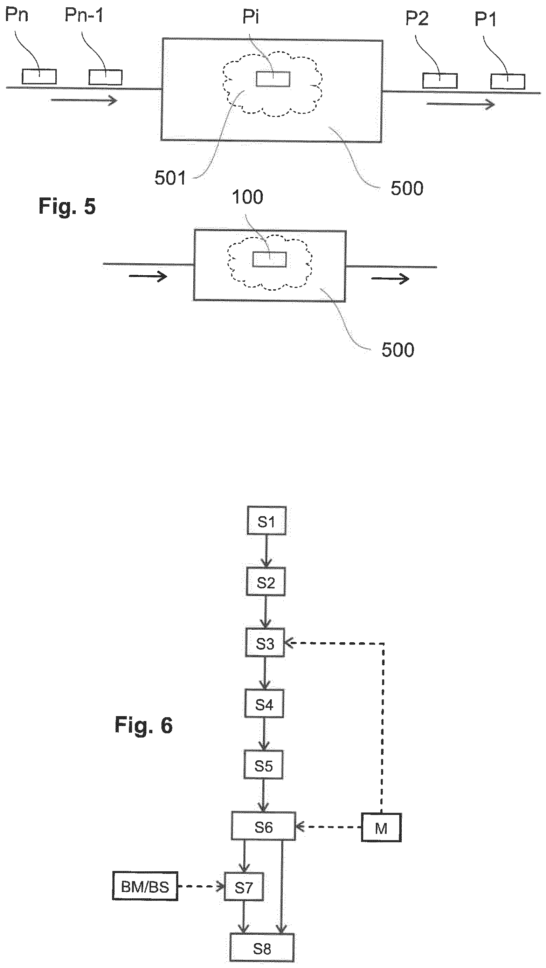

For instance, in an exemplary measurement f.i. taken by dual-energy X-ray inspection apparatus 500 (Fig. 5), all bone splitter mimics in region B were detected, while in region A only those with 1.5 mm and 2.0 mm thickness were detected, but not those with 1.0 mm thickness. This may correspond to a suitable detection resolution setting for the X-ray inspection apparatus 500. While the settings for the image evaluation algorithms of the X-ray inspection apparatus could theoretically be modified or improved to detect also the thinnest bone splitter mimic in the region (A) of longest propagation path length, such settings would, in use during X-ray inspection of a charge of real food products of the same product type lead to an alert/possible rejection with respect to too many products with respect to a due number. On the other hand side, if for an exemplary situation, a next verifying method of said X-ray inspection apparatus 500 after a given time interval would result in that f.i. the 1.5 mm thickness bone splitter mimic in region A and/or the 1.0 mm thickness of bone splitter mimic in region B would no more be detected, one can conclude in a deterioration of the detection capability. By setting suitable thresholds in this way, one may also determine when readjustment of the setting parameters of the X-ray inspection apparatus is required. Moreover, when using said phantom 300 in a plant comprising more than one X-ray inspection apparatus, relative comparison between them can be made on the basis of verifying process using said phantom, such as to detect differences in their detection capabilities which may be undesirable, and to have the possibility to readjust the detection capabilities all to the same level.

In Fig. 5, an X-ray inspection system with an X-ray inspection apparatus 500 is shown in a schematical manner. Products P1, P2, ... Pn are transported via known transporting device 501 to pass through the inspection zone 501 of dual-energy X-ray inspection apparatus 500 and are analyzed by means of dual X-ray inspection in a manner known in the art. For instance, a dual-energy X-ray apparatus of Eagle (Eagle Product Inspection) could be used, as well as other X-ray inspection apparatus commercially available on the market.

From time to time, when said X-ray inspection process is disactivated or interrupted, the X-ray inspection apparatus 500 performs X-ray inspection with one or more phantoms according to the present invention, f.i. one or more of phantoms 100, 200, 300, and 400, to verify the detection capability of the X-ray inspection apparatus 500 in accordance with the explanations given above.

In the flow diagram of Fig. 6, a preferred method of manufacturing phantoms according to the present invention is given. In step S1, a typical product sample of the product type in question is selected, f.i. product Pi of Fig. 5. In step S2, a X-ray image is generated from said product sample Pi.

In step S3, calculations as explained above are made to convert the X-ray image of step S2 to a thickness profile/height profile for the phantom. In step S4, the height profile is implemented in the form of a mold, created f.i. by 3D-printing technique, the mold corresponding to a negative of the phantom form. Then, it is understood that step S4 may comprise a smoothing of the height profile on a scale corresponding to a local roughness due to the typical X-ray random noise, whereafter the surface form of the height profile re-corresponds roughly to the surface form/roughness of the product sample Pi. In step S5, a mold, f.i. of silicon, is prepared according to said height profile. In step S6, a casting process for the mold with material M, in this embodiment f.i. an epoxy resin, is started. In (optional) step S7, casting is interrupted, bone mimics (BM) and/or bone splitter mimics (BS) are placed in selected zones (D, E, F / A, B) onto the cast material, and casting is continued afterwards, such as to embed said bone/bone splitter mimic into the cast material. In the last step S8, the phantom is removed from the mold after the cast material is sufficiently solidified. The dotted line from M to S3 indicates that a material property information, here the absorption coefficient of the epoxy resin, is input into the calculation for determining the global scaling factor.

It is understood that, for some aspects, it would not be necessary to reproduce/mimic the exact contour form of the original product. As an alternative, one could f.i. use only a part of the X-ray image, f.i. the part including regions A and B, to have a simpler contour form, f.i. a rectangular or rounded, f.i. elliptical contour form. Otherwise, one could also add to the X-ray image additional portions such as to arrive at a simplified contour form for the mold, or a combination of both variations.

Moreover, in region C, one recognizes pieces of still different material, here stainless steel (SS), which are spaced apart and have increased thickness starting from 0.6 mm, over 0.8 mm, 1.0 mm, 1.5 mm, 1.8 mm, to 2.0 mm. Such addition is purely optional, even the provision of several identical bone pieces (BS) in one or more regions (A, B) is only a preferred example of the invention, which is not limited by specific arrangements of exemplary embodiments.

## Claims

1. Method of verifying the detection capability of an X-ray inspection apparatus (500) with respect to a product type of in particular a food product, wherein a body is subject to X-rays propagating through the body in an inspection zone (501) of the X-ray inspection apparatus, wherein e the body is a phantom (100; 200; 300; 400) mainly made from artificial material and having a height profile comprising at least two regions (A, B) with different height level and thereby different propagation path lengths, their path length difference correlating with a difference of measured X-ray attenuation arising between regions of a product sample of said product type mimicked by the phantom which regions correspond to said regions (A, B) of the phantom, and wherein it is, based on a X-ray image generated by the X-ray inspection of the phantom, determined whether the detection capability of the verified X-ray inspection apparatus is within given set detection parameters.

2. Method according to claim 1, wherein the phantom material comprises a polymer material (M) as base material.

3. Method according to claim 2, wherein the phantom comprises locally a piece (BS; BM) of a different material than said polymer material, in particular a calcium containing material, in particular in the region having the longer prolongation path length.

4. Method according to claim 3, wherein the phantom comprises a plurality of pieces (BS) of different material, said plurality in particular comprising a first group of pieces varying in their dimension in propagation path direction, a second group of pieces varying in dimension and/or form transverse to the propagation path direction, and/or a third group of pieces varying in material composition.

5. Method according to claim 3 or 4, wherein at least a part of said locally arranged piece(s) is/are embedded in the base material.

6. Method according to any of the preceding claims, where the X-ray inspection apparatus is configured to perform dual-energy X-ray attenuation measurement.

7. Method according to any of the preceding claims, wherein the overall area shape of the product sample transverse to the propagation path is mimicked by the phantom.

8. Method according to any of the preceding claims, wherein the X-ray inspection is repeated on the same X-ray inspection machine after a time interval and/or such a method with the same phantom is additionally executed for another X-ray inspection apparatus of in particular the same apparatus type.

9. Method according to any of the preceding claims, wherein the settings of the X-ray inspection apparatus are adjusted in dependency of the result of the X-ray inspection of the phantom.

10. Method of X-ray inspection of products, in particular food products, of a given product type by means of an X-ray inspection apparatus being in particular configured to perform dual-energy X-ray attenuation measurement, **characterized in that** said X-ray inspection apparatus is made subject to a verifying method according to any of the preceding claims with respect to said given product type.

11. Method of manufacturing a phantom for use in a method according to any of claims 1 to 9, wherein an X-ray measurement for a product sample of said product type is made, the respective local measured X-ray attenuation is converted into a thickness profile having thickness variations correlated to respective X-ray attenuation variations of the product sample, and manufacturing the phantom with a thickness in dependency of said thickness profile, such that a height profile of the phantom corresponds to the thickness profile and has different height levels.

12. Method according to claim 11, comprising determining an average absorption coefficient by use of an average thickness of the measured product sample and an average over the intensity of the transmitted X-ray, and calculating a local thickness, in particular pixelwise with respect to the X-ray image, by means of the average absorption coefficient and the local intensity of the corresponding local X-ray intensity of the measured product sample, to obtain relative thickness differences.

13. Method according to claim 12, wherein determination of the thickness profile for the phantom involves a global scaling of said relative thickness variations in dependency of the absorption coefficient of the polymer material used as base material for the phantom.

14. Method according to any of claims 11 to 13, wherein the manufacturing of the phantom involves casting a polymer material into a mold, the mold form being created in particular on the basis of 3-D printing.

15. Method according to claim 14, wherein casting is divided in two or more steps and one or more pieces of a different material, in particular calcium containing material is inserted inbetween two casting steps.

16. Phantom mimicking a product type of an in particular food product in a verifying method according to any of the claims 1 to 9 and/or manufactured by a method according to any of the claims 11 to 15, the phantom having a height profile comprising at least two regions (A, B) with different height level and thereby wherein their path length difference correlates with a difference of measured X-ray attenuation arising between regions of a product sample of said product type mimicked by the phantom which regions correspond to said regions (A, B) of the phantom.

## Patentansprüche

1. Verfahren zum Verifizieren der Detektionsfähigkeit einer Röntgenprüfvorrichtung (500) bezüglich einer Produktart, insbesondere eines Lebensmittelprodukts, wobei ein Körper Röntgenstrahlen ausgesetzt wird, die sich durch den Körper in einer Prüfzone (501) des Röntgenprüfvorrichtungs ausbreiten, wobei der Körper ein Phantom (100; 200; 300; 400) ist, das hauptsächlich aus künstlichem Material hergestellt ist und ein Höhenprofil aufweist, das wenigstens zwei Bereiche (A, B) mit unterschiedlicher Höheniveau und damit unterschiedlicher Ausbreitungsweglängen umfasst, wobei deren Weglängendifferenz mit einer Differenz der gemessenen Röntgenabschwächung korreliert, die zwischen Bereichen einer Produktprobe der Produktart, die durch das Phantom nachgeahmt wird, entsteht, wobei diese Bereiche den Bereichen (A, B) des Phantoms entsprechen, und wobei basierend auf einem Röntgenbild, das durch die Röntgenprüfung des Phantoms erzeugt wird, bestimmt wird, ob die Detektionsfähigkeit der verifizierten Röntgenprüfvorrichtung innerhalb gegebener festgelegter Detektionsparameter liegt.

2. Verfahren nach Anspruch 1, wobei das Phantommaterial ein Polymermaterial (M) als Basismaterial umfasst.

3. Verfahren nach Anspruch 2, wobei das Phantom lokal ein Stück (BS; BM) aus einem anderen Material als dem Polymermaterial, insbesondere einem kalziumhaltigen Material, umfasst, insbesondere in dem Bereich mit der längeren Ausbreitungsweglänge.

4. Verfahren nach Anspruch 3, wobei das Phantom eine Vielzahl von Stücken (BS) aus unterschiedlichem Material umfasst, wobei die Vielzahl insbesondere eine erste Gruppe von Stücken umfasst, die in ihrer Dimension in Ausbreitungswegrichtung variieren, eine zweite Gruppe von Stücken, die in Dimension und/oder Form quer zur Ausbreitungswegrichtung variieren, und/oder eine dritte Gruppe von Stücken, die in der Materialzusammensetzung variieren.

5. Verfahren nach Anspruch 3 oder 4, wobei wenigstens ein Teil des/der lokal angeordneten Stücks/e in das Basismaterial eingebettet ist.

6. Verfahren nach einem der vorhergehenden Ansprüche, wobei die Röntgenprüfvorrichtung zur Durchführung einer Dual-Energie-Röntgenabschwächungsmessung konfiguriert ist.

7. Verfahren nach einem der vorhergehenden Ansprüche, wobei die Gesamtoberflächenform der Produktprobe quer zum Ausbreitungsweg durch das Phantom nachgeahmt wird.

8. Verfahren nach einem der vorhergehenden Ansprüche, wobei die Röntgenprüfung an derselben Röntgenprüfmaschine nach einem Zeitintervall wiederholt wird und/oder ein solches Verfahren mit demselben Phantom zusätzlich für eine andere Röntgenprüfvorrichtung, insbesondere desselben Vorrichtungtyps, ausgeführt wird.

9. Verfahren nach einem der vorhergehenden Ansprüche, wobei die Einstellungen der Röntgenprüfvorrichtung in Abhängigkeit vom Ergebnis der Röntgenprüfung des Phantoms angepasst werden.

10. Verfahren zur Röntgenprüfung von Produkten, insbesondere Lebensmittelprodukten, einer gegebenen Produktart mittels einer Röntgenprüfvorrichtung, die insbesondere zur Durchführung einer Dual-Energie-Röntgenabschwächungsmessung konfiguriert ist,
**dadurch gekennzeichnet, dass** die Röntgenprüfvorrichtung zu Teil eines Verifizierungsverfahrens nach einem der vorhergehenden Ansprüche bezüglich der gegebenen Produktart gemacht wird.

11. Verfahren zur Fertigung eines Phantoms zur Verwendung in einem Verfahren nach einem der Ansprüche 1 bis 9, wobei eine Röntgenmessung für eine Produktprobe der Produktart durchgeführt wird, die jeweilige lokal gemessene Röntgenabschwächung in ein Dickenprofil umgewandelt wird, das Dickenvariationen aufweist, die mit jeweiligen Röntgenabschwächungsvariationen der Produktprobe korrelieren, und das Phantom mit einer Dicke in Abhängigkeit von dem Dickenprofil gefertigt wird, so dass ein Höhenprofil des Phantoms dem Dickenprofil entspricht und unterschiedliche Höhenniveaus aufweist.

12. Verfahren nach Anspruch 11, umfassend das Bestimmen eines durchschnittlichen Absorptionskoeffizienten unter Verwendung einer durchschnittlichen Dicke der gemessenen Produktprobe und eines Durchschnitts über die Intensität der durchgelassenen Röntgenstrahlung, und das Berechnen einer lokalen Dicke, insbesondere pixelweise bezüglich des Röntgenbildes, mittels des durchschnittlichen Absorptionskoeffizienten und der lokalen Intensität der entsprechenden lokalen Röntgenintensität der gemessenen Produktprobe, um relative Dickenunterschiede zu erhalten.

13. Verfahren nach Anspruch 12, wobei die Bestimmung des Dickenprofils für das Phantom eine globale Skalierung der relativen Dickenvariationen in Abhängigkeit vom Absorptionskoeffizienten des als Basismaterial für das Phantom verwendeten Polymermaterials beinhaltet.

14. Verfahren nach einem der Ansprüche 11 bis 13, wobei die Fertigung des Phantoms das Gießen eines Polymermaterials in eine Form beinhaltet, wobei die Form insbesondere auf Basis des 3D-Drucks erstellt wird.

15. Verfahren nach Anspruch 14, wobei das Gießen in zwei oder mehr Schritten unterteilt ist und ein oder mehrere Stücke eines anderen Materials, insbesondere kalziumhaltiges Material, zwischen zwei Gießschritte eingefügt werden.

16. Phantom, das eine Produktart eines insbesondere Lebensmittelprodukts in einem Verifizierungsverfahren nach einem der Ansprüche 1 bis 9 nachahmt und/oder nach einem Verfahren nach einem der Ansprüche 11 bis 15 gefertigt wird, wobei das Phantom ein Höhenprofil aufweist, das wenigstens zwei Bereiche (A, B) mit unterschiedlichem Höhenniveau umfasst, und wobei deren Weglängendifferenz mit einer Differenz der gemessenen Röntgenabschwächung korreliert, die zwischen Bereichen einer Produktprobe der Produktart, die durch das Phantom nachgeahmt wird, entsteht, wobei diese Bereiche den Bereichen (A, B) des Phantoms entsprechen.

## Revendications

1. Procédé de vérification de la capacité de détection d'un appareil d'inspection à rayons X (500) pour un type de produit, en particulier un produit alimentaire, dans lequel un corps est soumis à des rayons X se propageant à travers le corps dans une zone d'inspection (501) de l'appareil d'inspection à rayons X, dans lequel le corps est un fantôme (100; 200; 300; 400) principalement constitué de matériau artificiel et présentant un profil de hauteur comprenant au moins deux régions (A, B) de hauteur différente et donc de longueurs de trajet de propagation différentes, leur différence de longueur de trajet étant corrélée à une différence d'atténuation des rayons X mesurée entre des régions d'un échantillon de produit dudit type de produit imité par le fantôme, lesquelles régions correspondent auxdites régions (A, B) du fantôme, et dans lequel l'image radiographique générée par l'inspection à rayons X du fantôme permet de déterminer le fait de savoir si la capacité de détection de l'appareil d'inspection à rayons X vérifié se situe dans des paramètres de détection définis.

2. Procédé selon la revendication 1, dans lequel le matériau fantôme comprend un matériau polymère (M) comme matériau de base.

3. Procédé selon la revendication 2, dans lequel le fantôme comprend localement un morceau (BS ; BM) d'un matériau différent de celui dudit matériau polymère, en particulier un matériau contenant du calcium, en particulier dans la région ayant la longueur de trajet de prolongation la plus longue.

4. Procédé selon la revendication 3, dans lequel le fantôme comprend une pluralité de pièces (BS) de matériaux différents, ladite pluralité comprenant notamment un premier groupe de pièces dont la dimension varie dans la direction du trajet de propagation, un deuxième groupe de pièces dont la dimension et/ou la forme varient transversalement à la direction du trajet de propagation, et/ou un troisième groupe de pièces dont la composition matérielle varie.

5. Procédé selon la revendication 3 ou 4, dans lequel au moins une partie de ladite/desdite(s) pièce(s) agencée(s) localement est/sont noyée(s) dans le matériau de base.

6. Procédé selon l'une quelconque des revendications précédentes, dans lequel l'appareil d'inspection à rayons X est configuré pour réaliser une mesure d'atténuation des rayons X à double énergie.

7. Procédé selon l'une quelconque des revendications précédentes, dans lequel la forme de la zone globale de l'échantillon de produit transversalement au trajet de propagation est imitée par le fantôme.

8. Procédé selon l'une quelconque des revendications précédentes, dans lequel l'inspection à rayons X est répétée sur la même machine d'inspection à rayons X après un intervalle de temps et/ou un tel procédé avec le même fantôme est également exécuté pour un autre appareil d'inspection à rayons X, en particulier du même type d'appareil.

9. Procédé selon l'une quelconque des revendications précédentes, dans lequel les réglages de l'appareil d'inspection à rayons X sont ajustés selon le résultat de l'inspection à rayons X du fantôme.

10. Procédé d'inspection à rayons X de produits, notamment de produits alimentaires, d'un type de produit donné au moyen d'un appareil d'inspection à rayons X étant notamment configuré pour réaliser une mesure d'atténuation des rayons X à double énergie,
**caractérisé en ce que** ledit appareil d'inspection à rayons X est soumis à un procédé de vérification selon l'une quelconque des revendications précédentes par rapport audit type de produit donné.

11. Procédé de fabrication d'un fantôme destiné à être utilisé dans un procédé selon l'une quelconque des revendications 1 à 9, dans lequel une mesure de rayons X pour un échantillon de produit dudit type de produit est réalisée, l'atténuation des rayons X mesurée locale respective est convertie en un profil d'épaisseur ayant des variations d'épaisseur corrélées aux variations d'atténuation des rayons X respectives de l'échantillon de produit, et la fabrication du fantôme avec une épaisseur dépendant dudit profil d'épaisseur, de telle sorte qu'un profil de hauteur du fantôme corresponde au profil d'épaisseur et présente différents niveaux de hauteur.

12. Procédé selon la revendication 11, comprenant la détermination d'un coefficient d'absorption moyen à l'aide d'une épaisseur moyenne de l'échantillon de produit mesuré et d'une moyenne sur l'intensité des rayons X transmis, et le calcul d'une épaisseur locale, en particulier au niveau des pixels par rapport à l'image radiographique, au moyen du coefficient d'absorption moyen et de l'intensité locale de l'intensité des rayons X locaux correspondants de l'échantillon de produit mesuré, pour obtenir des différences d'épaisseur relatives.

13. Procédé selon la revendication 12, dans lequel la détermination du profil d'épaisseur du fantôme implique une mise à l'échelle globale desdites variations d'épaisseur relative selon le coefficient d'absorption du matériau polymère utilisé comme matériau de base pour le fantôme.

14. Procédé selon l'une quelconque des revendications 11 à 13, dans lequel la fabrication du fantôme implique le moulage d'un matériau polymère dans un moule, la forme du moule étant créée notamment sur la base d'une impression 3D.

15. Procédé selon la revendication 14, dans lequel la coulée est divisée en deux ou plusieurs étapes et une ou plusieurs pièces d'un matériau différent, en particulier un matériau contenant du calcium, sont insérées entre deux étapes de coulée.

16. Fantôme imitant un type de produit d'un produit alimentaire particulier dans un procédé de vérification selon l'une quelconque des revendications 1 à 9 et/ou fabriqué par un procédé selon l'une quelconque des revendications 11 à 15, le fantôme ayant un profil de hauteur comprenant au moins deux régions (A, B) avec un niveau de hauteur différent et dans lequel leur différence de longueur de trajet est corrélée à une différence d'atténuation des rayons X mesurée survenant entre des régions d'un échantillon de produit dudit type de produit imité par le fantôme, lesquelles régions correspondent auxdites régions (A, B) du fantôme.
